# FASCICULE DE BREVET EUROPEEN

(11) **EP 0 727 445 B1**
(45) Date de publication et mention de la délivrance du brevet: **20.01.1999**
(21) Numéro de dépôt: 96400244.8
(22) Date de dépôt: 06.02.1996
(51) Int. Cl.: C08F 8/46, C08F 8/32, C07C 51/567

(54) **Procédé de fabrication d'anhydrides alkenyl ou polyalkenyls succiniques sans formation de résines**
Verfahren zur Herstellung von Alkenyl- und Polyalkenylbernsteinsäureanhydriden ohne Harzbildung
Process for the preparation of alkenyl and polyalkenyl succinic anhydrides free from resin formation

(30) Priorité: 15.02.1995 FR 9501815
(43) Date de publication de la demande: 21.08.1996
(73) Titulaire: INSTITUT FRANCAIS DU PETROLE, 92502 Rueil-Malmaison (FR)
(72) Inventeur: Binet, Daniel, F-78310 Maurepas (FR); Gateau, Patrick, F-78310 Maurepas (FR); Durand, Jean-Pierre, F-78400 Chatou (FR)

(56) Documents cités:
- EP-A- 0 457 599
- EP-A- 0 587 381
- WO-A-94/02571
- DE-A- 2 708 757
- DE-A- 4 319 672
- US-A- 5 041 622

## Description

La présente invention concerne un procédé de fabrication d'anhydrides d'acides alkényl ou polyalkénylsucciniques exempts de chlore.

Dans le domaine des additifs pour produits pétroliers, notamment des additifs détergents pour carburants moteurs et des dispersants pour lubrifiants moteurs, il est souvent fait appel, pour synthétiser les composés amphiphiles recherchés dans ces applications, à des polyisobutènes fonctionnalisés, le plus souvent à l'aide d'anhydride maléique. Ces anhydrides polyisobuténylsucciniques sont préparés suivant deux procédés principaux.

Le premier consiste en la condensation d'anhydride maléïque sur du polyisobutène suivant une réaction de ène-synthèse. Cette réaction nécessite une température élevée et des temps de réaction tels que, dans ces conditions, apparaissent des résines résultant de la polymérisation de l'anhydride maléique. Ces produits secondaires obligent à une étape de filtration, rendue très délicate par la consistance de ces produits. Des améliorations visant à diminuer la quantité de résines sont souvent proposées. Elles consistent le plus souvent à effectuer la réaction en présence d'inhibiteurs de polymérisation radicalaire, comme dans les documents DE-A-1 102 142, DE-A-3 320 468 et US-A-3 476 774 ; de composés halogénés comme dans les documents GB-A-1 356 802, GB-A 1 480 453 (= FR-A-2 273 014), US-A-3 960 900, DE-A-3 320 468 déjà cité, US-A-4 278 604, et US-A-4 255 340 ; de sels métalliques, comme dans les documents GB-A-2 081 274 et W0-82/00467 ; ou de peroxydes comme dans le document US-A-4 599 432 (= FR-A-2 555 595). Une autre méthode, décrite par exemple dans le document US-A-4 496 746 consiste à faire réagir l'anhydride maléique en émulsion dans un solvant inerte, ceci grâce à l'utilisation d'un tensio-actif. Dans tous les cas, les produits secondaires ne sont jamais totalement éliminés.

Dans un second type de procédé, on réalise d'abord une chloration du polyisobutène suivie de la condensation du polyisobutène chloré et d'anhydride maléique. Ce procédé concurrence le précédent grâce à un abaissement de la température de réaction, à une meilleure conversion et à l'absence de réactions secondaires conduisant à des résines insolubles. Toutefois, ce procédé conduit habituellement à des produits contenant une certaine quantité de chlore résiduel, ce qui les exclut d'un nombre d'applications de plus en plus important, compte tenu des spécifications de plus en plus sévères concernant les teneurs en chlore des additifs pour produits pétroliers. La présente invention propose un procédé de fabrication d'anhydrides alkényl ou polyalkénylsucciniques exempts de chlore, qui ne met pas en jeu de réaction en émulsion ou en suspension et dans lequel les produits secondaires sont totalement éliminés. Ce procédé évite ainsi une étape de filtration longue, délicate et coûteuse lors de la préparation d'additifs pour produits pétroliers exempts de chlore, notamment d'additifs détergents pour carburants moteurs et d'additifs dispersants sans cendres pour lubrifiants moteurs.

Le procédé de fabrication d'anhydrides alkényl ou polyalkénylsucciniques selon l'invention comprend la réaction dans des conditions de ène-synthèse entre une oléfine ou une polyoléfine et un anhydride d'acide dicarboxylique insaturé, et en solution dans au moins un solvant aromatique choisi parmi le toluène et les xylènes.

Les oléfines et polyoléfines mises en jeu peuvent avoir une masse moléculaire moyenne en nombre comprise entre 400 et 10 000 et de préférence entre 400 et 3 000. Les polyoléfines considérées consistent plus particulièrement en des polyisobutènes présentant une teneur en doubles liaisons "exo" supérieure à 50 %. Ils sont obtenus de préférence par polymérisation de l'isobutène en présence d'un catalyseur ne contenant pas de chlore, tel que par exemple le trifluorure de bore. L'anhydride d'acide carboxylique insaturé est choisi plus particulièrement dans le groupe formé par l'anhydride maléique et les anhydrides maléiques substitués par un ou deux groupes méthyles sur les atomes de carbone de la liaison éthylénique, à savoir l'anhydride citraconique (méthyl maléïque) et l'anhydride pyrocinchonique (diméthyl maléique). De préférence, on utilise l'anhydride maléique.

La caractéristique essentielle du procédé de l'invention est la mise en oeuvre de la réaction de ène-synthèse au sein d'un solvant aromatique choisi parmi le toluène et les xylènes. La teneur en solvant est plus particulièrement comprise entre 35 et 60 % en masse, par exemple 50 % en masse du milieu réactionnel.

Par ailleurs, la réaction est conduite à une température comprise entre 150 et 300° C, de préférence entre 180 et 250° C, par exemple sous pression autogène. En général, la pression développée lors de la réaction est comprise entre 2 et 40 bars, plus particulièrement entre 3 et 10 bars.

L'anhydride maléique ou maléique substitué est en général utilisé dans un rapport molaire de 0,5 à 2, de préférence de 1 à 1,2, par rapport à l'oléfine ou à la polyoléfine (le plus souvent le polyisobutène).

Les anhydrides alkényl et polyalkénylsucciniques (en particulier polyisobuténylsucciniques) sont obtenus par le procédé de l'invention, avec des rendements élevés, qui peuvent atteindre 85 %. A titre indicatif, dans le cas des anhydrides polyisobuténylsucciniques dérivant de polyisobutène de masse molaire d'environ 1000, de tels rendements correspondent à des indices d'anhydride allant jusqu'à environ 0,08 mole d'anhydride pour 100 g de produit.

Les anhydrides alkénylsucciniques et polyalkénylsucciniques, en particulier polyisobutènylsucciniques, obtenus par le procédé de l'invention sont des produits intermédiaires pour la fabrication d'additifs détergents pour carburants moteurs ou d'additifs dispersants sans cendres pour lubrifiants moteurs.

Pour cette fabrication, les anhydrides sont modifiés, de manière connue, notamment par réaction avec des amines pour former des imides.

Les amines utilisées sont plus particulièrement des polyéthylène polyamines telles que la triéthylène tétramine, ou la tétraéthylène pentamine, utilisées par exemple en une proportion molaire de 0,5 à 1 par rapport à l'anhydride alkényl - ou polyalkénylsuccinique (en particulier polyisobuténylsuccinique).

Les exemples suivants illustrent l'invention, les exemples de préparation 1, 5, 7 et 8 étant donnés à titre de comparaison.

### Exemple 1 (comparatif)

Dans un autoclave équipé d'un système d'agitation, on introduit :
250 g de polyisobutène de masse moléculaire moyenne en nombre voisine de 1000 et dont la composition des doubles liaisons terminales, déterminée par RMN du proton est la suivante :
- 90 % de doubles liaisons "exo" ;
- 10 % de doubles liaisons "endo" ;
- et 24,5 g d'anhydride maléique.

Le mélange est dégazé par balayage d'azote à température ambiante. Il est ensuite agité pendant 16 heures à 200° C. L'anhydride maléique n'ayant pas réagi est ensuite éliminé par distillation sous vide à 150° C. Après dilution dans 275 g de toluène, le mélange réactionnel est filtré afin de séparer les produits secondaires formés, qui représentent une fois séchés 0,55 g soit 0,2 % en masse des produits mis en oeuvre. Le toluène est éliminé par distillation sous vide pour conduire à un produit dont l'indice d'anhydride (nombre de moles d'anhydride pour 100 g de produit) est de 0,071.

### Exemple 2

Dans un autoclave équipé d'un système d'agitation, on introduit :
250 g de polyisobutène de masse moléculaire moyenne en nombre voisine de 1000 et dont la composition des doubles liaisons terminales, déterminée par RMN du proton est la suivante :
- 90 % de doubles liaisons "exo" ;
- 10 % de doubles liaisons "endo" ;
- et 24,5 g d'anhydride maléique
- et 147,8 g de xylènes, soit 35% en masse du milieu réactionnel.

Le mélange est dégazé par balayage d'azote à température ambiante. Il est ensuite agité pendant 15 heures à 200° C. La pression développée lors de la réaction est de 2,2 bars. Aucun produit secondaire n'est formé. Les xylènes et l'anhydride maléique n'ayant pas réagi sont éliminés par distillation sous vide à 150°C. L'indice d'anhydride du produit obtenu (nombre de moles d'anhydride pour 100 g de produit) est de 0,067.

### Exemple 3

Si, dans l'exemple 1, toutes choses étant égales par ailleurs, la réaction est conduite en présence de 274,5 g de xylènes, soit 50% en masse du milieu réactionnel, aucun produit secondaire n'est formé. La pression développée lors de la réaction est de 3 bars. Après distillation des xylènes et élimination sous vide à 150°C de l'anhydride maléique libre, on obtient un produit ayant un indice d'anhydride de 0,065.

### Exemple 4

Si, dans l'exemple 3, la température de réaction est portée à 220° C, on ne constate aucune formation de résines. La pression développée pendant la réaction est alors de 5 bars. Le produit obtenu a un indice d'anhydride de 0,075.

### Exemple 5 (comparatif)

Si dans l'exemple 1, toutes choses étant égales par ailleurs, la réaction est conduite en présence de 91,5 g de xylènes, soit 25% en masse du milieu réactionnel, on constate la formation de produits secondaires huileux et bruns. La présence de ces produits insolubles interdit toute utilisation du produit sans purification.

### Exemple 6

Si dans l'exemple 3 les xylènes sont remplacés par une quantité équivalente de toluène, la pression atteinte lors de la réaction est de 6,2 bars et aucun produit secondaire n'est formé. Le produit obtenu après distillation du solvant et élimination sous vide de l'anhydride maléique n'ayant pas réagi a un indice d'anhydride de 0,067.

### Exemple 7 (comparatif)

Si dans l'exemple 3, toutes choses étant égales par ailleurs, on remplace les xylènes par une coupe aromatique ayant un point initial de distillation de 186° C et un point final de 214° C, le produit obtenu, après élimination sous vide du solvant et de l'anhydride maléique non réagi, contient 0,3 % en masse de composés insolubles dont l'analyse infra-rouge montre les caractères acide et aromatique. Après élimination des composés insolubles, le produit a un indice d'anhydride de 0,044.

### Exemple 8 (comparatif)

A 100 g du produit obtenu dans l'exemple 1 après filtration et élimination du toluène, on ajoute 109,6 g de la coupe aromatique utilisée dans l'exemple 5 et 9,6 g de tétraéthylène pentamine. Le mélange réactionnel ainsi obtenu est agité pendant 6 heures à 165° C. Le mélange obtenu a une teneur en azote de 1,8 % en masse.

### Exemple 9

A 100 g du produit obtenu dans l'exemple 3, on ajoute 110,1 g de la coupe aromatique utilisée dans l'exemple 5 et 10,1 de tétraethylène pentamine. Le mélange réactionnel ainsi obtenu est agité pendant 6 heures à 165° C. Le produit final a une teneur en azote de 1,65 % en masse.

Afin d'évaluer les performances d'un produit synthétisé suivant l'invention, comparativement à celles d'un produit issu d'une ène-synthèse classique, on a réalisé deux séries d'essais moteurs (moteurs Diesel et moteur à essence).

### Exemple 10 : Test sur moteur Diesel

Un test d'encrassement des injecteurs sur moteur XUD9 est effectué. Le carburant utilisé est un gazole dont les caractéristiques sont détaillées dans le Tableau 1. La tendance à l'encrassement est donnée par le débit résiduel moyen des quatre injecteurs, obtenu après 6 heures de fonctionnement du moteur. Les essais sont réalisées comparativement au gazole seul, en additivant celui-ci avec 350 ppm des solutions obtenues dans les exemples 8 et 9 (soit 250 ppm de matière active).

**Tableau 1**

| | |
|---|---|
| Masse volumique | 837,2 kg/m³ |
| Teneur en soufre | 0,045 % masse |
| Distillation | |
| Point initial | 204,5° C |
| 5 % | 240,0° C |
| 10 % | 253,0° C |
| 20 % | 269,5° C |
| 30 % | 280,5° C |
| 40 % | 287,5° C |
| 50 % | 293,0° C |
| 60 % | 298,0° C |
| 70 % | 303,5° C |
| 80 % | 309,5° C |
| 90 % | 321,0° C |
| 95 % | 336,5° C |
| Point final | 349,0° C |

Les résultats sont rassemblés dans le Tableau 2 et montrent l'efficacité comparable des produits selon l'invention avec ceux issus d'une ène-synthèse classique.

**Tableau 2**

| **Résultats d'encrassement d'injecteurs (XUD9 - 6 heures)** | | | |
|---|---|---|---|
| Levée d'aiguille (mm) | | | |
| | | | |
| Débit résiduel moyen (%) | 0,1 | 0,2 | 0,3 |
| Gazole (référence) | 14,7 | 23,9 | 33,4 |
| Gazole + 500 ppm d'additif de l'exemple 8 | 31,2 | 41,1 | 51,7 |
| Gazole + 500 ppm d'additif de l'exemple 9 | 36,4 | 47,2 | 57,6 |

### Exemple 11 : Test sur moteur à essence

Des formulations à base des additifs des exemples 8 et 9 et de 50 parties de polypropylène glycol de masse moléculaire voisine de 1000 sont préparées. Des essais moteurs sont réalisés afin d'évaluer l'influence des additifs sur les quantités de dépôts au niveau des soupapes d'admission.

La méthode d'essai utilise un moteur Mercedes M 102 E et elle fonctionne de façon cyclique selon un schéma porte à porte durant 60 heures. Le carburant de référence utilisé est un carburant sans plomb ayant un indice d'octane "Recherche" de 96,8. Les essais sont menés en présence de 750 ppm des formulations à tester et comparées à un essai en l'absence d'additif. Les résultats obtenus (tableau 3) sont exprimés en poids (grammes) de dépôts sur les tulipes des soupapes d'admission. Ils montrent l'efficacité comparable d'un additif issu de l'invention avec l'efficacité d'un produit obtenu à partir d'un anhydride polyisobuténylsuccinique préparé selon une méthode classique.

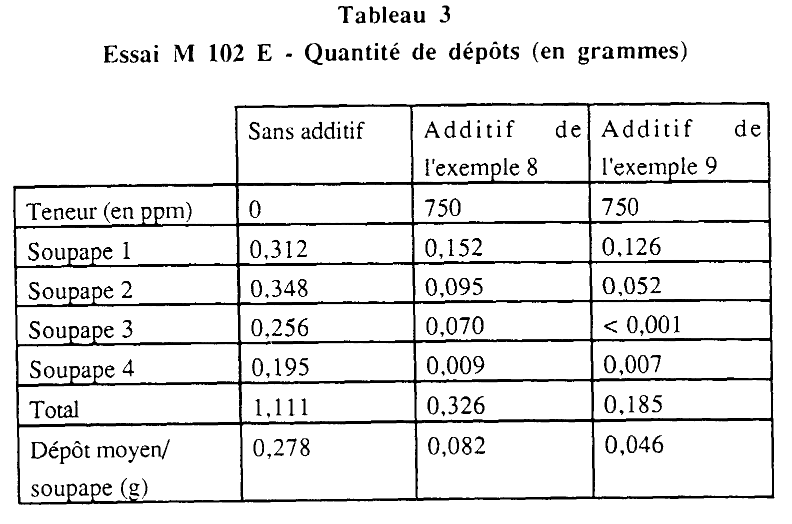

## Revendications

1. Procédé de préparation d'anhydride alkényl ou polyalkénylsuccinique par réaction de éne-synthèse entre au moins une oléfine ou une polyoléfine et au moins un anhydride d'acide dicarboxylique insaturé choisi parmi l'anhydride maléique et les anhydrides maléiques substitués par 1 ou 2 groupes méthyles, caractérisé en ce que ladite réaction est réalisée au sein d'au moins un solvant aromatique choisi parmi le toluène et les xylènes utilisé en une proportion de 35 à 60 % en masse par rapport au milieu réactionnel.

2. Procédé selon la revendication 1, caractérisé en ce que ladite oléfine ou polyoléfine a une masse moléculaire moyenne en nombre comprise entre 400 et 10 000.

3. Procédé selon la revendication 1, caractérisé en ce que ladite oléfine ou polyoléfine a une masse moléculaire moyenne en nombre comprise entre 400 et 3 000.

4. Procédé selon l'une des revendications 1 à 3, caractérisé en ce que ladite polyoléfine est un polyisobutène présentant une teneur en doubles liaisons "exo" supérieure à 50 %.

5. Procédé selon l'une des revendications 1 à 4, caractérisé en ce que la réaction est conduite à une température de 150 à 300°C.

6. Procédé selon l'une des revendications 1 à 4, caractérisé en ce que la réaction est conduite à une température de 180 à 250°C.

7. Procédé selon l'une des revendications 1 à 6, caractérisé en ce que l'anhydride maléique ou maléique substitué est mis en jeu dans un rapport molaire de 0,5 à 2 avec l'oléfine ou la polyoléfine.

8. Procédé selon l'une des revendications 1 à 6, caractérisé en ce que l'anhydride maléique ou maléique substitué est mis en jeu dans un rapport molaire de 1 à 1,2 avec l'oléfine ou la polyoléfine.

9. Un anhydride alkényl ou polyalkénylsuccinique obtenu par un procédé selon l'une des revendications 1 à 8.

10. Un anhydride polyisobuténylsuccinique obtenu par un procédé selon l'une des revendications 1 à 8 par réaction entre un polyisobutène à teneur en doubles liaisons "exo" supérieure à 50 % et l'anhydride maléique.

11. Le produit de la réaction d'au moins un anhydride polyisobuténylsuccinique selon la revendication 10 avec au moins une polyéthylène polyamine.

12. Produit selon la revendication 10, caractérisé en ce que la polyéthylène polyamine est mise en jeu en une proportion molaire de 0,5 à 1 par rapport à l'anhydride polyisobuténylsuccinique.

## Claims

1. A process for manufacturing an alkenyl anhydride or polyalkenylsuccinic anhydride under ene-synthesis reaction conditions between at least one olefin or polyolefin and at least one unsatured dicarboxylic anhydride selected from maleic anhydride and maleic anhydrides substituted by 1 or 2 methyl groups, characterized in that said reaction is carried out in at least one aromatic solvent selected from toluene and xylenes, employed at a ratio of 35 to 60 % by weight relative to the reaction medium.

2. A process according to claim 1, characterized in that said olefin or polyolefin has a number average molecular weight of 400 to 10,000.

3. A process according to claim 1, characterized in that said olefin or polyolefin has a number average molecular weight of 400 to 3000.

4. A process according to any of claims 1 to 3, characterized in that said polyolefin is a polyisobutene that has a content of external double bonds that is greater than 50 %.

5. A process according to any of claims 1 to 4, characterized in that the reaction is carried out at a temperature of 150 to 300 °C.

6. A process according to any of claims 1 to 4, characterized in that the reaction is carried out at a temperature of 180 to 250 °C.

7. A process according to any of claims 1 to 6, characterized in that the maleic anhydride or substituted maleic anhydride is used in a molar ratio of 0.5/1 to 2/1 with respect to the olefin or the polyolefin.

8. A process according to any of claims 1 to 6, characterized in that the maleic anhydride or substituted maleic anhydride is used in a molar ratio of 1/1 to 1.2/1 with respect to the olefin or the polyolefin.

9. An alkenyl anhydride or polyalkenylsuccinic anhydride that is obtained by a process according to any of claims 1 to 8.

10. A polyisobutenylsuccinic anhydride that is obtained by a process according to any of claims 1 to 8, by reaction between a polyisobutene with a content of external double bonds that is greater than 50 % and maleic anhydride.

11. The product obtained by the reaction of at least one polyisobutenylsuccinic anhydride according to claim 10 with at least one polyethylene polyamine.

12. A product according to claim 11, characterized in that polyethylene polyamine is used at a molar ratio of 0.5/1 to 1/1 relative to the polyisobutenylsuccinic anhydride.

## Patentansprüche

1. Verfahren zur Herstellung von Alkenyl- oder Polyalkenylbernsteinsäureanhydrid durch eine En-Synthese-Reaktion zwischen mindestens einem Olefin oder Polyolefin und mindestens einem ungesättigten Dicarbonsäureanhydrid, ausgewählt aus der Gruppe Maleinsäureanhydrid und der durch eine oder zwei Methylgruppen substituierten Maleinsäureanhydride, dadurch gekennzeichnet, daß die genannte Reaktion in mindestens einem aromatischen Lösungsmittel, ausgewählt aus der Gruppe Toluol und der Xylole durchgeführt wird, das in einem Mengenanteil von 35 bis 60 Massenprozent, bezogen auf das Reaktionsmedium, verwendet wird.

2. Verfahren nach Anspruch 1, dadurch gekennzeichnet, daß das genannte Olefin oder Polyolefin eine zahlendurchschnittliche Molekularmasse zwischen 400 und 10 000 hat.

3. Verfahren nach Anspruch 1, dadurch gekennzeichnet, daß das genannte Olefin oder Polyolefin eine zahlendurchschnittliche Molekularmasse zwischen 400 und 3000 hat.

4. Verfahren nach einem der Ansprüche 1 bis 3, dadurch gekennzeichnet, daß das genannte Polyolefin ein Polyisobuten ist, das einen Gehalt an "Exo"-Doppelbindungen von mehr als 50 % aufweist.

5. Verfahren nach einem der Ansprüche 1 bis 4, dadurch gekennzeichnet, daß die Reaktion bei einer Temperatur von 150 bis 300°C durchgeführt wird.

6. Verfahren nach einem der Ansprüche 1 bis 4, dadurch gekennzeichnet, daß die Reaktion bei einer Temperatur von 180 bis 250°C durchgeführt wird.

7. Verfahren nach einem der Ansprüche 1 bis 6, dadurch gekennzeichnet, daß das Maleinsäureanhydrid oder substituierte Maleinsäureanhydrid in einem Molverhältnis von 0,5 bis 2, bezogen auf das Olefin oder das Polyolefin, eingesetzt wird.

8. Verfahren nach einem der Ansprüche 1 bis 6, dadurch gekennzeichnet, daß das Maleinsäureanhydrid oder das substituierte Maleinsäureanhydrid in einem Molverhältnis von 1 bis 1,2, bezogen auf das Olefin oder das Polyolefin, eingesetzt wird.

9. Alkenyl- oder Polyalkenyl-bernsteinsäureanhydrid, das nach einem Verfahren gemäß einem der Ansprüche 1 bis 8 hergestellt worden ist.

10. Polyisobutenyl-bernsteinsäureanhydrid, das nach einem Verfahren gemäß einem der Ansprüche 1 bis 8 hergestellt worden ist durch Umsetzung zwischen einem Polyisobuten mit einem Gehalt an "Exo"-Doppelbindungen von > 50 % mit Maleinsäureanhydrid.

11. Produkt der Umsetzung zwischen mindestens einem Polyisobutenylbernsteinsäureanhydrid nach Anspruch 10 und mindestens einem Polyethylenpolyamin.

12. Produkt nach Anspruch 11, dadurch gekennzeichnet, daß das Polyethylenpolyamin in einem Molverhältnis von 0,5 bis 1, bezogen auf das Polyisobutenyl-bernsteinsäureanhydrid, eingesetzt wird.
